(19)

Europäisches
Patentamt
European
Patent Office
Office européen
des brevets

(11) **EP 2 770 175 A1**

(12) **EUROPEAN PATENT APPLICATION**

published in accordance with Art. 153(4) EPC

(43) Date of publication:
**27.08.2014 Bulletin 2014/35**

(21) Application number: **11874225.3**

(22) Date of filing: **20.10.2011**

(51) Int Cl.:
***F01M 11/10*** *(2006.01)*

(86) International application number:
**PCT/JP2011/005894**

(87) International publication number:
**WO 2013/057768 (25.04.2013 Gazette 2013/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(71) Applicant: **TOYOTA JIDOSHA KABUSHIKI
KAISHA
Toyota-shi, Aichi-ken, 471-8571 (JP)**

(72) Inventor: **ITO, Yusuke
Toyota-shi, Aichi-ken, 471-8571 (JP)**

(74) Representative: **Albutt, Anthony John
D Young & Co LLP
120 Holborn
London EC1N 2DY (GB)**

(54) **OIL DETERIORATION DETERMINATION DEVICE FOR INTERNAL COMBUSTION ENGINE**

(57)     To provide an oil degradation determining device for an internal combustion engine, which is able to determine degradation of oil accurately regardless of a driving state of a vehicle.

An EFI-ECU 41 estimates an amount of sludge precursor accumulated in an oil pan 4 based on temperature of an engine 1 and an injection quantity. Particularly, the EFI-ECU 41 calculates an amount of fuel injected in a combustion chamber 7 of the engine 1 per certain detection period including detection time, estimates an amount of sludge precursor accumulated in the oil pan 4 based on a calculated value of the accumulation amount of sludge precursor per unit amount of fuel, which is calculated based on current temperature of the engine 1, and a calculated value of the amount of fuel injected in the detection period from previous detection time until current detection time, and judges that engine oil O is degraded when the accumulation amount of the sludge precursor becomes a certain value or above.

FIG.1

EP 2 770 175 A1

## Description

TECHNICAL FIELD

The present invention relates to an oil degradation determining device for an internal combustion engine, and particularly to an oil degradation determining device for an internal combustion engine, which determines that oil circulating in the internal combustion engine is degraded.

BACKGROUND ART

In an engine mounted on a vehicle such as an automobile, respective sliding areas such as a piston, a crankshaft, and a connecting rod are lubricated and cooled by engine oil that circulates in the engine, but the engine oil is degraded with the use of the engine.

An additive is mixed in the engine oil in order to provide performance of engine oil, and, because the additive is consumed as the engine oil is used, the performance of engine oil provided is deteriorated.

Also, dirt is mixed into the engine oil with the use of the engine. As a result, the engine oil is degraded, and it becomes difficult for the engine oil to function as a lubricant, so it is necessary to change the engine oil.

Sludge is one of phenomena that occur when oil is degraded. As a producing process of sludge, sludge precursor (precursor of sludge) is first produced when hydrocarbon having a structure of olefin, aromatics or the like inside fuel is combusted in a combustion chamber.

The sludge precursor is captured mainly by engine oil that lubricates a piston inside the combustion chamber. The sludge precursor captured by the engine oil falls into an oil pan through between a cylinder and the piston.

Meanwhile, about several tens of presents of an additive is added to the engine oil in order to provide performance of engine oil.

There are various types of additive components, and, for example, there is polyisobutenyl succinimide-based ashless dispersant as an additive component that provides engine oil with dispersion and cleaning performance.

Also, there is metal-based cleaner such as Ca phenate, Ca sulfonate, Ca salicylate, or the like, as an additive component that provides acid neutralization and cleaning performance especially when temperature is high.

When the above-mentioned sludge precursor is mixed into engine oil, sludge precursor is dispersed in the engine oil by the ashless dispersant.

However, as deterioration of dispersion performance of engine oil advances with the use of the engine, and an amount of sludge precursor captured in the engine oil is increased in accordance with a driving state, the sludge precursor is polymerized with each other, and becomes an insoluble component with a large molecular weight. The insoluble component is aggregated or precipitated, and becomes sludge.

A reaction of the insoluble component being aggregated or precipitated is promoted by an acid substance, especially nitric acid, that is generated by a reaction between water, which is produced as moisture generated by combustion inside the engine is cooled, and NOx (nitrogen oxide) and SOx (sulfur oxide) contained in blowby gas that passes through between the cylinder and the piston.

Therefore, the engine oil is provided with an acid neutralization ability so as to inhibit a reaction between an acid substance, especially nitric acid, generated by a reaction with NOx (nitrogen oxide) and SOx (sulfur oxide), and the insoluble component, so that formation of sludge due to aggregation and precipitation of the insoluble component is restrained.

Therefore, as sludge precursor is increased inside the engine oil, the insoluble component is also increased, and sludge is produced when the dispersion performance and the acid neutralization performance of the engine oil are exceeded.

As a thing that changes engine oil at appropriate time in consideration of performances of engine oil, an oil degradation determining device is known, which gives warning for encouraging change of engine oil once a driving state such as travel distance and traveling period of a vehicle reaches an upper limit that is set in anticipation of a certain level of safety factor in consideration of the performances of engine oil, after the change of the engine oil (for example, see Patent Document 1).

Citation List

Patent Document

Patent Document 1: Japanese Patent Application Publication 2004-156455 A (JP 2004-156455 A)

SUMMARY OF THE INVENTION

Problem to be Solved by the Invention

However, with such a conventional oil degradation determining device, it is likely that engine oil still having sufficient lubrication performance is changed at an early point depending on a driving state, or engine oil is changed after the engine oil is degraded, so an engine can be contaminated.

This means that sludge precursor is produced mainly by combustion of fuel, and has a property of being easily dissolved in engine oil and collection efficiency into engine oil becomes higher as temperature is decreased.

In other words, for example, a driver who has many opportunities to drive at low temperature of an engine, an amount of sludge precursor collected in engine oil is increased compared to a driver who has many opportunities to drive at high temperature of an engine, and there is a possibility that sludge is formed relatively early.

Therefore, engine oil is likely to be degraded before a travel distance and a traveling period (a duration) of a vehicle after engine oil change reaches an upper limit.

The present invention has been accomplished in order to solve the above-mentioned conventional problem, and an object is to provide an oil degradation determining device for an internal combustion engine, which is able to accurately determine degradation of oil regardless of a driving state of a vehicle.

Means for Solving the Problem

In order to achieve the above-mentioned object, an oil degradation determining device for an internal combustion engine according to the present invention is an oil degradation determining device for an internal combustion engine, which determines degradation of oil circulating in the internal combustion engine, and is structured by an oil degradation determining device having oil degradation determination means that determines degradation of oil by estimating an amount of sludge precursor accumulated in the oil storage means based on temperature of the internal combustion engine and an injection quantity.

Sludge precursor is a kind of impurities mixed into oil, and becomes a raw material of sludge. Sludge precursor is polymerized with each other and becomes an insoluble component with a large molecular weight, and the insoluble component is aggregated or precipitated, thus becoming sludge.

Sludge precursor before being the insoluble component is produced mainly by combustion of fuel, and has a property of being easily dissolved in oil and having higher collection efficiency into oil as temperature is decreased.

The sludge precursor is a primary product of fuel, and performance of oil has nothing to do with the producing process.

Based on the property of sludge precursor, the oil degradation determining device for the internal combustion engine is able to accurately determine degradation of oil regardless of a driving state such as a travel distance of a vehicle and a duration after oil change, by determining degradation of oil by estimating an amount of sludge precursor accumulated in the oil storage means based on temperature of the internal combustion engine and an injection quantity. Thus, it is possible to encourage a driver to carry out oil change at appropriate time.

Preferably, the oil degradation determination means of the oil degradation determining device may be structured by oil degradation determination means that calculates an amount of fuel injected inside a cylinder of the internal combustion engine per certain detection period including detection time, and estimates an amount of sludge precursor accumulated in the oil storage means based on a calculated value of an accumulation amount of sludge precursor per unit amount of fuel, which is calculated based on current temperature of the internal combustion engine, and a calculated value of an amount of fuel injected in the detection period from previous detection time until current detection time.

This oil degradation determining device estimates an amount of sludge precursor accumulated in the oil storage means based on the calculated value of the accumulation amount of sludge precursor per unit amount of fuel, which is calculated based on current temperature of the internal combustion engine, and a calculated value of the amount of fuel injected in the detection period from previous detection time until current detection time. Therefore, it is possible to estimate an accumulation amount of sludge precursor accurately, and determine degradation of oil highly accurately. Thus, it is possible to encourage a driver to carry out oil change at appropriate time.

Preferably, the oil degradation determination means of the oil degradation determining device may be structured by oil degradation determination means that determines that oil is degraded on condition that an accumulation amount of sludge precursor has become a certain value or above.

Since this oil degradation determining device determines that oil is degraded on condition that the accumulation amount of sludge precursor has become a certain value or above, it is possible to determine degradation of oil accurately by eliminating the need for setting an oil change period in accordance with a driving state such as travel distance of a vehicle and duration after oil change, and it is possible to encourage a driver to carry out oil change at appropriate time.

Preferably, the oil degradation determining device may be structured by an oil degradation determining device, in which temperature detection means that detects temperature of the internal combustion engine is included, and the oil degradation determination means calculates the accumulation amount of sludge precursor per unit amount of fuel based on detection information of the temperature detection means.

In this oil degradation determining device, since the temperature detection means detects temperature of the internal combustion engine, and the accumulation amount of sludge precursor per unit amount of fuel is calculated based on detection information of the temperature detection means, it is possible to estimate the accumulation amount of sludge precursor in accordance with a load and the like on the internal combustion engine, in other words, in accordance with an actual driving state. Therefore, it is possible to determine degradation of oil accurately regardless of a driving state, such as a travel distance of a vehicle and a duration after oil change.

Preferably, the oil degradation determining device may be structured by an oil degradation determining device, in which warning means is provided, which warns that oil has been degraded based on a driving signal inputted form the oil degradation determination means, and the oil degradation determination means outputs the driving signal to the warning means on condition that an accumulation amount of sludge precursor has become a certain value or above.

In this oil degradation determining device, since the warning means is able to warn a driver when oil degradation is determined, it is possible to ensure that a driver is encouraged to carry out oil change at appropriate time.

Preferably, the oil degradation determining device may be structured by an oil degradation determining device, in which oil changing period setting means is included, which sets an upper limit of a changing period of oil stored in the oil storage means, and the oil degradation determination means determines that oil is degraded on condition that an accumulation amount of the sludge precursor has become a certain value or above before the upper limit of the changing period set by the oil changing period setting means is reached.

In a case of a driver who has many opportunities to drive in a driving state with a small load on the internal combustion engine, for example, to drive at low temperature of the internal combustion engine, an amount of sludge precursor collected in oil is large, compared to a driver who has many opportunities to drive in a driving state with a large load on the internal combustion engine, for example, to drive at high temperature of the internal combustion engine, and there is a possibility that sludge is formed relatively early. Therefore, oil is likely to be degraded before reaching the upper limit of the changing period of oil in the oil storage means, and the internal combustion engine is contaminated,

In the present invention, since it is determined that oil is degraded on condition that the accumulation amount of sludge precursor has become a certain value or above before the upper limit of the changing period set by the oil changing period setting means is reached, it is possible to determine oil degradation before the upper limit of the changing period set by the oil changing period setting means is reached, in a case of a situation where there are many opportunities to drive at a driving state of the internal combustion engine at low temperature, and a large amount of sludge precursor is collected in oil, and so on.

Thus, it is possible to estimate the accumulation amount of sludge precursor in accordance with an actual driving state of the internal combustion engine, and it is possible to determine oil degradation accurately regardless of a driving state such as a duration after oil change.

Preferably, the oil degradation determining device may be structured by an oil degradation determining device, in which travel distance setting means is included, which sets an upper limit of a travel distance of a vehicle, and the oil degradation determination means determines that oil is degraded on condition that the accumulation amount of sludge precursor has become a certain value or above before the upper limit of the travel distance set by the travel distance setting means is reached.

This way, it is possible to estimate the accumulation amount of sludge precursor in accordance with an actual driving state of the internal combustion engine, and it is possible to determine oil degradation accurately regardless of a driving state such as a travel distance of a vehicle.

Advantageous Effects of Invention

According to the present invention, it is possible to provide an oil degradation determining device for an internal combustion engine, which is able to determine oil degradation accurately regardless of a driving state of a vehicle.

BRIEF DESCRIPTION OF THE DRAWINGS

[FIG. 1] FIG. 1 is a view showing an embodiment of an oil degradation determining device for an internal combustion engine according to the present invention, and is a schematic structural view of an engine provided with the oil degradation determining device for the internal combustion engine;
[FIG. 2] FIG. 2 is a view showing the embodiment of the oil degradation determining device for the internal combustion engine according to the present invention, and is a configuration diagram of a system that controls the engine;
[FIG. 3] FIG. 3 is a view showing the embodiment of the oil degradation determining device for the internal combustion engine according to the present invention, and is a view showing a sludge precursor accumulation map; and
[FIG. 4] FIG. 4 is a view showing the embodiment of the oil degradation determining device for the internal combustion engine according to the present invention, and is a flowchart of oil degradation determination processing.

MODES FOR CARRYING OUT THE INVENTION

An embodiment of an oil degradation determining device for an internal combustion engine according to the present invention is explained below by using the drawings.

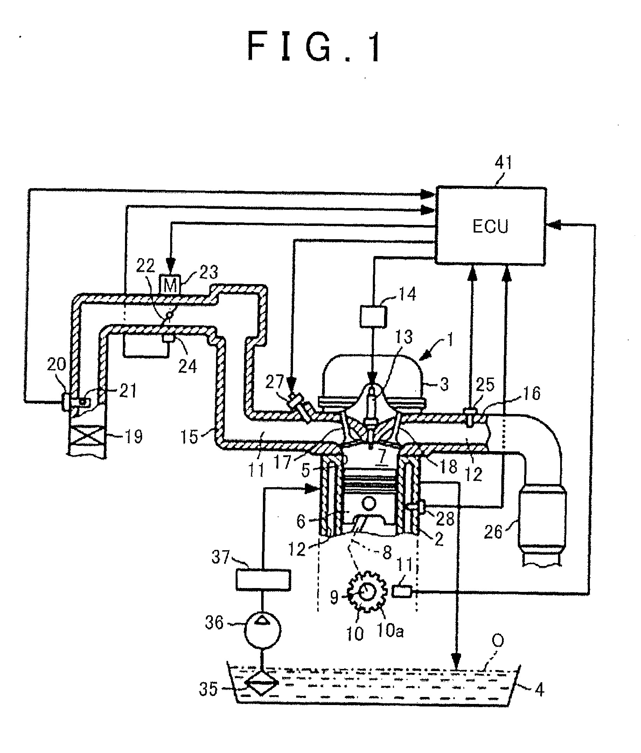

FIG. 1 to FIG. 4 are views showing an embodiment of the oil degradation determining device for the internal combustion engine according to the present invention.

First of all, a structure is explained.

In FIG. 1, an engine 1 serving as an internal combustion engine is structured by including a cylinder block 2, a cylinder head 3 fastened to an upper portion of the cylinder block 2, and an oil pan 4 which is fastened to a lower portion of the cylinder head 3 and stores engine oil O.

In the cylinder block 2, a cylinder bore 5 that structures a cylinder is formed, and a piston 6 is housed in the cylinder bore 5. Also, a combustion chamber 7 is formed in an upper portion of the cylinder bore 5, and the combustion chamber 7 is structured by a space surrounded by the cylinder bore 5, a top surface of the piston 6, and a lower surface of the cylinder head 3.

The engine 1 is a so-called four cycle gasoline engine, which carries out a series of four processes that are an intake process, a compression process, an expansion process, and an exhaust process, while the piston 6 reciprocates inside the cylinder bore 5.

The engine 1 of this embodiment is, for example, an in-line four cylinder engine having four cylinder bores 5 and four pistons 6. Note that the number of cylinders is an example, and is not limited to four cylinders.

The piston 6 is connected with a crankshaft 9 through a connecting rod 8, and a reciprocating motion of the piston 6 is converted into a rotation motion of the crankshaft 9 by the connecting rod 8.

In this embodiment, the oil pan 4 structures oil storage means, and the engine oil O stored in the oil pan 4 is pumped up by an oil pump 36 through an oil strainer 35 that removes foreign matters when the engine 1 is operated.

The engine oil pumped up by the oil pump 36 is supplied to sliding areas such as the piston 6, the crankshaft 9, and the connecting rod 8 after being purified by an oil filter 37, and is used for lubrication, cooling and so on of the sliding areas.

The engine oil supplied to the sliding areas is recovered in the oil pan 4 after being used for lubrication, cooling and so on of the sliding areas, and is stored in the oil pan 4 until being pumped up again by the oil pump 36.

The oil pump 36 is mechanically joined to the crankshaft 9, and is structured by a mechanical oil pump that discharges engine oil to an oil passage by using driving power of the engine 1. Note that this oil pump may be structured by an electric pump that is driven independently from actuation of the engine 1. However, FIG. 1 schematically shows a circulation path of the engine oil O.

Further, a water jacket 12 is provided in the cylinder block 2 around the cylinder bore 5, and cooling water cooled by a non-illustrated radiator is introduced into this water jacket 12.

The cooling water introduced into the water jacket 12 is returned to the radiator again after cooling the cylinder bore 5, and cooling water cooled by the radiator is flown back to the water jacket 12 again.

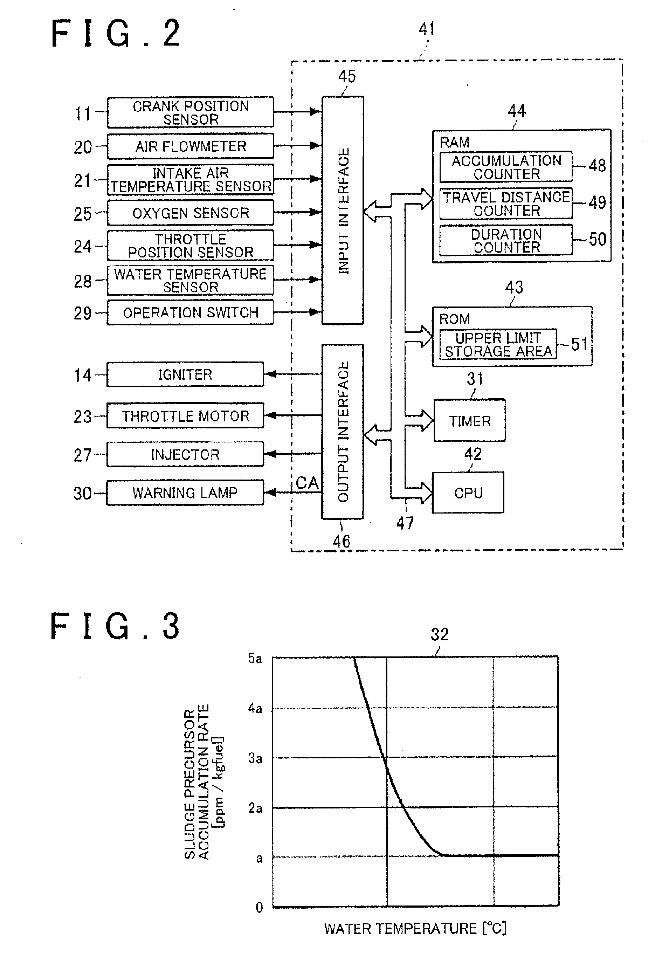

A spark plug 13 is provided in the cylinder head 3 of the engine 1, and ignition time of the spark plug 13 is adjusted by an igniter 14 (see FIG. 2).

An intake pipe 15 and an exhaust pipe 16 is connected to the cylinder head 3, and the intake pipe 15 and the exhaust pipe 16 are communicated with the combustion chamber 7. An intake valve 17 is provided between the intake pipe 15 and the combustion chamber 7, and the intake pipe 15 and the combustion chamber 7 are communicated with and blocked from each other by driving the intake valve 17 to open and close.

Also, an exhaust valve 18 is provided between the exhaust pipe 16 and the combustion chamber 7, and the exhaust pipe 16 and the combustion chamber 7 are communicated with and blocked from each other by driving the exhaust valve 18 to open and close.

The opening and closing drive of the intake valve 17 and the exhaust valve 18 is carried out respectively by rotation of a non-illustrated intake camshaft and exhaust camshaft to which rotation of the crankshaft 9 is transmitted.

Meanwhile, the intake pipe 15 is provided with an air cleaner 19, a hot-wire air flowmeter 20, an intake air temperature sensor 21, and an electronically controlled throttle valve 22 for adjusting an intake air mass of the engine 1.

The throttle valve 22 is driven by a throttle motor 23, and opening of the throttle valve 22 is detected by a throttle position sensor 24.

The exhaust pipe 16 is provided with an oxygen sensor 25 that detects an oxygen concentration in exhaust gas, and a three-way catalyst 26 that purifies exhaust gas.

The intake pipe 15 is provided with an injector 27 for fuel injection. Fuel is supplied at given pressure to the injector 27 by a fuel pump from a fuel tank, and the injector 27 injects fuel to the intake pipe 15.

Fuel injected to the intake pipe 15 is mixed with intake air, becomes air-fuel mixture, and is introduced into the combustion chamber 7, and the air-fuel mixture introduced into the combustion chamber 7 is combusted and exploded as ignited by the spark plug 13.

Due to combustion and explosion of air-fuel mixture inside the combustion chamber 7, the piston 6 reciprocates, and the crankshaft 9 rotates.

A signal rotor 10 having a plurality of teeth 10a on an outer periphery is attached to the crankshaft 9, and a crank position sensor 11 is arranged near a side of the signal rotor 10.

The crank position sensor 11 is, for example, an electromagnetic pickup, and generates a pulse-like signal (output pulse) corresponding to the tooth 10 of the signal rotor 10 when the crankshaft 9 rotates.

Further, a water temperature sensor 28 serving as temperature detection means for detecting engine water temperature (cooling water temperature) is arranged in the cylinder block 2 of the engine 1.

In this embodiment, the spark plug 13 and the injector 27 are controlled based on an output signal of an EFI (electronic fuel injection)-ECU (electronic control unit) 41.

Further, the EFI-ECU 41 executes oil degradation control for determining degradation of the engine oil O inside the oil pan 4.

As shown in FIG. 2, the ECU 41 is structured by including a CPU (central processing unit) 42, a ROM (read only memory) 43, a RAM (random access memory) 44, an input interface 45, an output interface 46 and so on.

The ROM 43 stores various types of control programs including an oil degradation determination control program, maps that are referred to when these various types of control programs are executed, and so on.

The CPU 42 executes various types of computing processing including the oil degradation determination processing based on various types of control programs and maps stored in the ROM 43. The RAM 44 is a memory that temporarily stores computation results in the CPU 42 and data inputted from the various types of sensors mentioned above, and is a nonvolatile memory that structures a part of a work area.

The CPU 42, the ROM 43, and the RAM 44 are connected with each other through a bus 47, and also connected to the input interface 45 and the output interface 46.

The crank position sensor 11, the air flowmeter 20, the intake air temperature sensor 21, the oxygen sensor 25, the throttle position sensor 24, the water temperature sensor 28, an operation switch 29, and the so on are connected to the input interface 45.

The operation switch 29 is a switch operated by a driver when changing the engine oil O. The operation switch 29 is installed at a position where a driver is able to operate the operation switch 29, for example, in an upper portion of an instrument panel inside a vehicle interior.

The operation switch 29 is normally in an off state, and outputs an on signal to the EFI-ECU 41 in accordance with an operation by a driver. In a case where the EFI-ECU 41 recognizes the on signal of the operation switch 29, current date and time are stored in a storage area of the RAM 44 as oil changing information.

The current date and time may be obtained from time data of a non-illustrated navigation device connected to the EFI-ECU 41 by an in-vehicle network such as a CAN (controller area network), and time data of FM multiplex broadcasting received through a radio antenna.

The igniter 14 of the spark plug 13, the throttle motor 23 of the throttle valve 22, the injector 27, a warning lamp 30 for warning a driver and so on that the engine oil O is degraded and changing time has arrived, and so on are connected with the output interface 46.

The EFI-ECU 41 executes the oil degradation determination processing together with the various types of controls of the engine 1 based on output signals of the above-mentioned various types of sensors.

The EFI-ECU 41 according to this embodiment structures oil degradation determination means for determining degradation of the engine oil O by estimating an amount of sludge precursor accumulated in the oil pan 4, based on temperature of the engine 1 and an injection quantity injected from the injector 27.

In the ROM 43 of the EFI-ECU 41, a sludge precursor accumulation map 32 shown in FIG. 3 is stored, and, in the sludge precursor accumulation map 32, temperature of the engine 1, in other words, cooling water temperature, and an accumulation rate of sludge precursor are associated with each other.

The sludge precursor is produced by combustion of injected fuel, and has a property of being easily dissolved in engine oil and increasing collection efficiency into engine oil as temperature is decreased. Based on this relationship, the EFI-ECU 41 of this embodiment associates cooling water temperature with an accumulation rate of sludge precursor, in other words, an accumulation amount of sludge precursor per unit amount of fuel, stores the association as the sludge precursor accumulation map 32 in the ROM 43.

The EFI-ECU 41 refers to the sludge precursor accumulation map 32, and obtains an accumulation amount of sludge precursor per unit amount of fuel corresponding to cooling water temperature obtained from the water temperature sensor 28.

The EFI-ECU 41 calculates an amount of fuel injected in the cylinder bore 5 per certain detection period including detection time.

Here, the period in this embodiment indicates an interval from given time until next time, and the time indicates a point in time.

The EFI-ECU 41 computes a base injection quantity based on an intake air mass obtained from the air flowmeter 20, and engine speed obtained from the crank position sensor 11, carries out correction computation for correcting the base injection quantity in accordance with cooling water temperature obtained from the water temperature sensor 28 and throttle opening obtained from the throttle position sensor 24, and decides an actual injection quantity injected from the injector 27.

Therefore, the EFI-ECU 41 calculates a total injection quantity injected in a certain period between current detection time and previous detection time, and calculates an accumulation amount of sludge precursor produced from the total injection quantity. An amount of sludge precursor produced with respect to an injection quantity may be calculated by using a calculation formula.

In order to obtain time information, that is the certain period of time, time may be measured by a timer 31 provided in the EFI-ECU 41, and a duration may be obtained based on time data of a navigation device or FM multiplex broadcasting. In this embodiment, the CPU 43 obtains time information by using the timer 31.

The EFI-ECU 41 estimates an amount of sludge precursor accumulated in the oil pan 4 based on a calculated value of an accumulation amount of sludge precursor per unit amount of fuel, which is calculated based on current cooling water temperature obtained from the above-mentioned sludge precursor accumulation map 32, and a calculated value of an amount of fuel injected in a detection period from previous detection time until current detection time.

An accumulation counter 48, which stores an accumulation amount of sludge precursor by adding an amount of sludge precursor produced, is allocated to a storage area of the RAM 44, and the EFI-ECU 41 determines that engine oil O circulating the engine 1 is degraded on condition that an accumulation amount of sludge precursor accumulated by the accumulation counter 48 has become a certain value or above, and outputs a driving signal CA to the warning lamp 30.

The warning lamp 30 is installed at a position where the warning lamp 30 is visible to a driver, for example, in an instrument such as a speedometer, tachometer, and the like on an instrument panel.

The warning lamp 30 warns a driver that engine oil O is degraded by being lighted or blinking based on the driving signal CA inputted from the EFI-ECU 41. In this embodiment, the warning lamp 30 structures warning means.

Note that, instead of the warning lamp 30, a speaker or the like that gives warning by voice may be used as the warning means, or a buzzer or the like that gives warning by sound may be used as the warning means.

Further, a travel distance counter 49 that measures a travel distance of a vehicle after changing the engine oil O, and a duration counter 50 that measures a duration after changing the engine oil O are allocated to the storage area of the RAM 44.

The CPU 42 measures a duration after change of the engine oil 0 based on time information of the timer 31, and stores the duration in the duration counter 50. Also, the CPU 42 measures a travel distance of a vehicle after change of the engine oil O based on detection information of the crank position sensor 11, and stores the travel distance in the travel distance counter 49.

Further, upper limits are set for count values of the travel distance counter 49 and the duration counter 50, and the EFI-ECU 41 outputs the driving signal CA to the warning lamp 30 on condition that the count value of the travel distance counter 49 has reached the upper limit, or the count value of the duration counter 50 has reached the upper limit.

The upper limits of the count values of the travel distance counter 49 and the duration counter 50 are stored in an upper limit storage area 51 in the ROM 43.

The warning lamp 30 warns a driver that the engine oil O is degraded by being lighted or blinking based on the driving signal CA inputted from the EFI-ECU 41.

Further, the EFI-ECU 41 outputs the driving signal CA to the warning lamp 30 in a case where it is determined that an accumulation amount of sludge precursor has become a certain value or above, before the count value of the travel distance counter 49 reaches the upper limit or the count value of the duration reaches the upper limit.

In this embodiment, the RAM 44 having the travel distance counter 49, and the ROM 43 having the upper limit storage area 51 structure travel distance setting means. Further, the RAM 44 having the duration counter 50, and the ROM 43 having the upper limit storage area 51 structure oil changing period setting means.

Next, effects are explained.

As a producing process of sludge, sludge precursor, which is precursor of sludge, is produced when hydrocarbon having a structure of olefin, aromatics or the like inside fuel is combusted in the combustion chamber.

The sludge precursor is captured by engine oil that lubricates the piston 6, mainly because a part of fuel inside the combustion chamber is not completely combusted, and the structure is changed due to an oxidization reaction.

The sludge precursor captured by the engine oil falls into the oil pan 4 through between the cylinder bore 5 and the piston 6.

Since about several tens of presents of additive is added to the engine oil O in order to provide performance of the engine oil O,

There are various types of additive components, and, there is, for example, polyisobutenyl succinimide-based ashless dispersant as an additive component that provides the engine oil O with dispersion and cleaning performance.

Also, there is, for example, metal-based cleaner such as Ca phenate, Ca sulfonate, Ca salicylate, as an additive component that provides acid neutralization and cleaning performance especially when temperature is high.

When the sludge precursor is mixed into the engine oil O, the sludge precursor is dispersed into the engine oil O by the ashless dispersant.

However, as deterioration of dispersion performance of the engine oil O advances in accordance with the use of the engine 1, and the sludge precursor captured in the engine oil O is increased in accordance with a driving state, the sludge precursor is polymerized with each other, and becomes an insoluble component with a large molecular weight. Once this insoluble component is aggregated or precipitated, the insoluble component becomes sludge.

Here, a reaction of the insoluble component being aggregated or precipitated is promoted by an acid substance, especially nitric acid, that is generated by a reaction between water, which is produced as moisture generated inside the engine 1 by combustion is cooled, and NOx (nitrogen oxide) and SOx (sulfur oxide) contained in blowby gas that passes through between the cylinder and the piston.

Therefore, by providing the engine oil O with an acid neutralization ability, a reaction between an acid substance, especially nitric acid, which is generated by a reaction with NOx (nitrogen oxide) and SOx (sulfur oxide), and the insoluble component is inhibited, so as to restrain formation of sludge as the insoluble component is aggregated and precipitated.

As stated above, the sludge precursor before becoming the insoluble component is produced mainly by combustion of fuel, and has a property of being easily dissolved in the engine oil O and increasing collection efficiency into the engine oil O as temperature is decreased.

The sludge precursor is a primary product of fuel, and performance of the engine oil O has nothing to do with the producing process.

In this embodiment, based on the property of the sludge precursor, degradation of the engine oil O is determined by estimating an amount of sludge precursor accumulated in the oil pan 4 based on temperature of the engine 1 and an injection quantity. Thus, deterioration of the engine oil O is determined regardless of a driving state such as a travel distance of a vehicle and a duration after change of the engine oil, and performance of the engine oil O.

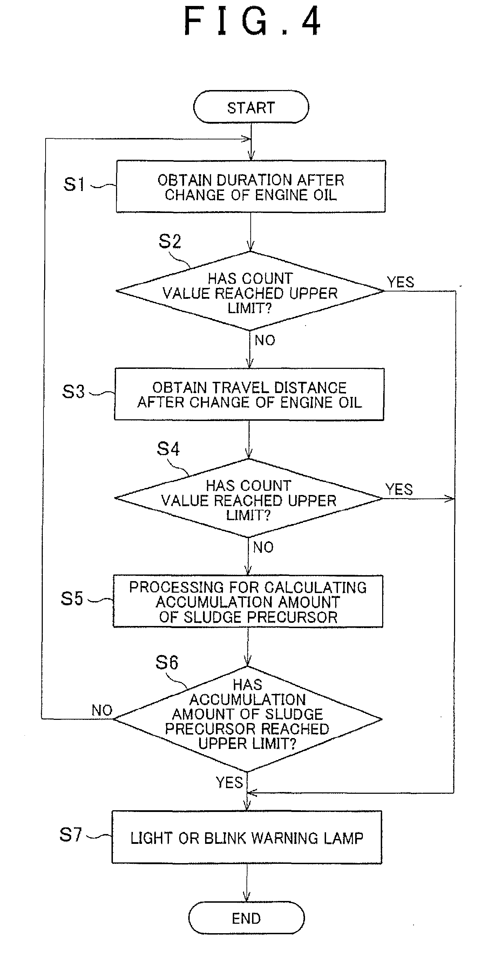

FIG. 4 is a flowchart of the oil degradation determination control program for executing the oil degradation determination processing, and the oil degradation determination control program is executed by the CPU 42.

First of all, the CPU 42 obtains a duration after changing the engine oil O based on a count value of the duration counter 50 (step S1).

The CPU 42 distinguishes whether or not the count value of the duration counter 50 has reached an upper limit (for example, 365 days) (step S2), and, in a case where it is determined that the count value has reached the upper limit, the CPU 42 outputs the driving signal CA to the warning lamp 30 to light or blink the warning lamp 30 (step S7), and ends the current processing.

Once the warning lamp 30 is lighted or blinked, a driver is going to carry out a work for changing the engine oil O inside the oil pan 4. Once the engine oil O inside the oil pan 4 is changed, and the operation switch 29 is operated by a driver, an on signal is outputted form the operation switch 29 to the EFI-ECU 41.

In a case where the CPU 42 of the EFI-ECU 41 recognizes the on signal of the operation switch 29, the CPU 42 resets the count values of the travel distance counter 49, the duration counter 50, and the accumulation counter 48.

Then, the CPU 42 stores current date and time in the duration counter 50 as engine oil changing information, and executes initialization processing to reset the count value of the travel distance counter 49 and the count value of the accumulation counter 48 to zero.

Meanwhile, when it is judged that the count value of the duration counter 50 is less than the upper limit, the CPU 42 obtains a travel distance after change of the engine oil O based on the count value of the travel distance counter 49 (step S3).

The CPU 42 distinguishes whether or not the count value of the travel distance counter 49 has reached an upper limit (for example, 50000 km) (step S4), and, when it is judged that the count value has reached the upper limit, the CPU 42 outputs the driving signal CA to the warning lamp 30 to light or blink the warning lamp 30 (step S7), and ends the current processing.

Once the warning lamp 30 is lighted or blinked, a driver is going to carry out a work for changing the engine oil O inside the oil pan 4. Once the engine oil O inside the oil pan 4 is changed, and the operation switch 29 is operated by the driver, an on signal is outputted from the operation switch 29 to the EFI-ECU 41.

In a case where the CPU 42 of the EFI-ECU 41 recognizes the on signal of the operation switch 29, the CPU 42 resets the count values of the travel distance counter 49, the duration counter 50, and the accumulation counter 48, and executes the above-mentioned initialization processing.

Meanwhile, when it is judged that the count value of the travel distance counter 49 is less than the upper limit, the CPU 42 executes processing for calculating an accumulation amount of sludge precursor (step S5).

In the processing for calculating the accumulation amount of the sludge precursor, the CPU 42 calculates an amount of fuel injected from the injector 27 in a detection period from previous detection time until current detection time based on time information from the timer 31.

Next, temperature of the engine 1 is detected based on detection information of the water temperature sensor 28, and temperature of sludge precursor captured in the engine oil is estimated from the temperature of the engine 1.

Next, the CPU 42 refers to the sludge precursor accumulation map 32 in FIG. 3, and obtains a sludge precursor accumulation amount Y per unit amount of fuel corresponding to the temperature of the engine 1 based on the detection information of the water temperature sensor 28.

Further, the CPU 42 calculates a fuel amount Q injected from the injector 27 in a detection period T from the previous detection time and the current detection time.

The CPU 42 calculates an accumulation amount AC of sludge precursor accumulated in the engine oil O in the detection period T, following an equation (1) below based on the sludge precursor accumulation amount Y and the fuel amount Q in the detection period T.

$$AC = Y \times Q \ \text{.........} (1)$$

By adding the accumulation amount AC of the sludge precursor to the accumulation counter 48, the CPU 42 is able to grasp the total accumulation amount of sludge precursor accumulated in the engine oil O per certain period of time, in other words, per detection period T.

Every time the CPU 42 executes the processing for calculating the accumulation amount of the sludge precursor, the CPU 42 distinguishes whether or not the accumulation amount of the sludge precursor accumulated in (added to) the accumulation counter 48 has reached the upper limit that is a certain value (step S6).

In a case where the CPU 42 judges that the accumulation amount of sludge precursor accumulated in the accumulation counter 48 has not reached the upper limit, the CPU 42 moves the processing to step S1. Further, in a case where the CPU 42 judges that the accumulation amount of sludge precursor accumulated in the accumulation counter 48 has reached the upper limit, the CPU 42 outputs the driving signal CA to the warning lamp 30 to light or blink the warning lamp 30 (step S7), and ends the current processing.

Once the warning lamp 30 is lighted or blinked, the driver is going to carry out a work for changing the engine oil O inside the oil pan 4. Note that once the engine oil O inside the oil pan 4 is changed, and the operation switch 29 is operated by a driver, an on signal is outputted from the operation switch 29 to the EFI-ECU 41.

In a case where the CPU 42 of the EFI-ECU 41 recognizes the on signal of the operation switch 29, the CPU 42 resets the count values of the travel distance counter 49, the duration counter 50, and the accumulation counter 48, and executes the above-mentioned initialization processing.

As stated so far, in the oil degradation determining device of this embodiment, the CPU 42 of the EFI-ECU 41 calculates an amount of fuel injected in the combustion chamber 7 of the engine 1 per certain detection period including detection time, estimates an amount of sludge precursor accumulated in the oil pan 4 based on the calculated value of the accumulation amount of sludge precursor per unit amount of fuel, which is calculated based on current temperature of the engine 1, and a calculated value of an amount of fuel injected in the detection period from pervious detection time until the current detection time, and judges that the engine oil O is deteriorated when the accumulation amount of the sludge precursor has become a certain value or above.

Thus, the CPU 42 is able to determine deterioration of the engine oil O based on the accumulation amount of the sludge precursor, which is a primary product of fuel, and the producing process of which has nothing to do with performance of the engine oil O.

Hence, the CPU 42 is able to determine deterioration of the engine oil O accurately. As a result, it is possible to encourage a driver to carry out change of the engine oil O at appropriate time.

Further, the oil degradation determining device according to this embodiment has the water temperature sensor 28 that detects temperature of the engine 1, and the CPU 42 calculates an accumulation amount of sludge precursor per unit amount of fuel based on detection information of the water temperature sensor 28.

Therefore, it is possible to estimate an accumulation amount of sludge precursor in accordance with an actual driving state like temperature of the engine 1, in other words, a load and so on applied to the engine 1, and it is possible to determine deterioration of the engine oil O accurately regardless of a driving state such as a travel distance of a vehicle and a duration after change of the engine oil.

Further, since the oil degradation determining device of this embodiment has the warning lamp 30 that warns that the engine oil O is degraded based on the driving signal CA inputted from the EFI-ECU 41, it is possible to ensure that a driver is encouraged to carry out change of the engine oil O at appropriate time.

The oil degradation determining device of this embodiment is provided with the RAM 44 having the duration counter 50 for measuring a duration after change of the engine oil O, and the travel distance counter 49 for measuring a travel distance of a vehicle after change of the engine oil O, and the ROM 43 having the upper limit storage area 51, as means for setting a changing period of the engine oil O stored in the oil pan 4.

Then, the CPU 42 determines that the engine oil O is deteriorated on condition that the count values of the accumulation amount of sludge precursor in the accumulation counter 48 has become the upper limit or above before reaching the upper limits of the travel distance and the duration set by the upper limit storage area 51 of the ROM 43, so the CPU 42 is able to determine deterioration of the engine oil O before reaching the upper limits of the duration of the engine oil O and the travel distance after change of the engine oil O.

In other words, in a case of a driver who has many opportunities to drive in a driving state with a small load on the engine 1, for example, to drive at low temperature of the engine 1, an amount of sludge precursor collected in the engine oil 0 is large compared to a driver who has many opportunities to drive in a driving state with a large load on the engine 1, for example, to drive at high temperature of the engine 1, and there is a possibility that sludge is formed relatively early.

Therefore, the engine oil O is likely to be degraded before reaching the upper limits of a duration of the engine oil O in the oil pan 4, and the travel distance after change of the engine oil, and the engine 1 is contaminated.

The CPU 42 of this embodiment is structured so as to determine that engine oil O is degraded on condition that the accumulation amount of the sludge precursor has become a certain value or above before reaching the upper limits of the upper limits of a duration and a travel distance set by the upper limit storage area 51.

Therefore, it is possible to determine degradation of the engine oil O before reaching the upper limits of a duration and a travel distance, in a case of a situation where there are many opportunities to drive in a driving state of the engine 1 at low temperature, and a large amount of sludge precursor is collected in the engine oil 0, and so on.

Thus, it is possible to estimate an accumulation amount of sludge precursor in accordance with an actual driving state of the engine 1, and it is possible to determine degradation of the engine oil O accurately regardless of a driving state such as a travel distance of a vehicle and a duration after change of the engine oil O.

Moreover, since it is possible to estimate an accumulation amount of the sludge precursor in accordance with an actual driving state of the engine 1, it is possible to extend the upper limit of the duration, and increase the upper limit of the travel distance, thereby enabling to give a margin to the duration and the travel distance.

In addition to this, it is possible to notify a user or a dealer of an optimum change time in accordance with a driving state even though a user such a driver, or a dealer does not strictly manages time for carrying out change of the engine oil O.

Further, in a case where a duration has reached the upper limit because a vehicle is left without being driven for a long period of time, the CPU 42 judges that the engine oil O has had oxidization degradation, and gives a warning by using the warning lamp 30, so it is possible to encourage a driver to carry out change of the engine oil O at appropriate time.

In this embodiment, although the temperature detection means is structured by the water temperature sensor 28, and temperature of the engine 1 is detected based on the water temperature sensor 28, an oil temperature sensor may be provided in the oil pan 4 as the temperature detection means, and the temperature of the engine 1 may be detected by the oil temperature sensor.

Alternatively, a sensor for directly detecting temperature of the cylinder bore 5 may be attached to the cylinder bore 5 as the temperature detection means, and temperature of the engine 1 may be detected by this sensor.

Further, a load on the engine 1 may be estimated based on information inputted from at least one or more sensors including the crank position sensor 11, the air flowmeter 20, the intake air temperature sensor 21, the throttle position sensor 24, the oxygen sensor 25, and so on, and temperature of the engine 1 may be estimated in accordance with the load on the engine 1.

In this case, a map may be used, in which detection information from the above-mentioned respective sensors, and temperature of the engine 1 are associated with each other.

In the oil degradation determining device for the internal combustion engine of this embodiment, the example was explained that the oil degradation determining device is applied to the internal combustion engine for a vehicle. However, the oil degradation determining device is applicable to anything that uses an internal combustion engine as a source of power, and, is also applicable to not only an internal combustion engine mounted on, for example, a so-called hybrid car, and a motorcycle, but also an internal combustion engine mounted on anything other than a vehicle, such as a ship and a construction machine.

As stated so far, the oil degradation determining device for the internal combustion engine according to the present invention has an effect of being able to determine oil degradation accurately regardless of a driving state of a vehicle, and is useful as an oil degradation determining device for an internal combustion engine, and so on, which determines degradation of oil that circulates in the internal combustion engine.

Description of Reference Numerals

1      engine (internal combustion engine)
4      oil pan (oil storage means)
5      cylinder bore (cylinder)
28     water temperature sensor (temperature detection means)
30     warning lamp (warning means)
41     EFI-ECU (oil degradation determination means)
43     ROM (oil changing period setting means, travel distance setting means)
44     RAM (oil changing period setting means, travel distance setting means)
O      engine oil (oil)

**Claims**

1. An oil degradation determining device for an internal combustion engine, which determines degradation of oil that circulates in the internal combustion engine, **characterized by** comprising oil degradation determination means that determines oil degradation by estimating an amount of sludge precursor accumulated in the oil storage means based on temperature of the internal combustion engine and an injection quantity.

2. The oil degradation determining device according to claim 1, **characterized in that**
the oil degradation determination means calculates an amount of fuel injected in a cylinder of the internal combustion engine per certain detection period including detection time, and
estimates an amount of sludge precursor accumulated in the oil storage means based on a calculated value of an accumulation amount of sludge precursor per unit amount of fuel, which is calculated based on current temperature of the internal combustion engine, and a calculated value of the amount of fuel injected in the detection period from previous detection time until current detection time.

3. The oil degradation determining device according to claim 1 or claim 2, **characterized in that** the oil degradation determination means determines that oil is degraded on condition that the accumulation amount of sludge precursor has become a certain value or above.

4. The oil degradation determining device according to any one of claim 1 to claim 3, **characterized by** comprising temperature detection means that detects temperature of the internal combustion engine, wherein

the oil degradation determination means calculates the accumulation amount of sludge precursor per unit amount of fuel based on detection information of the temperature detection means.

5. The oil degradation determining device according to claim 3 or claim 4, **characterized by** comprising warning means that warns that oil is degraded based on a driving signal inputted from the oil degradation determination means, wherein

the oil degradation determination means outputs the driving signal to the warning means on condition that the accumulation amount of sludge precursor has become a certain value or above.

6. The oil degradation determining device according to any one of claim 3 to claim 5, **characterized by** comprising oil changing period setting means that sets an upper limit of a changing period of oil stored in the oil storage means, wherein

the oil degradation determination means determines that oil is degraded on condition that the accumulation amount of sludge precursor has become a certain value or above before the upper limit of the changing period set by the oil changing period setting means is reached.

7. The oil degradation determining device according to any one of claim 3 to claim 5, **characterized by** comprising travel distance setting means that sets an upper limit of a travel distance of a vehicle, wherein the oil degradation determination means determines that oil is degraded on condition that the accumulation amount of sludge precursor has become a certain value or above before the upper limit of the travel distance set by the travel distance setting means is reached.

# F I G . 1

# FIG.2

CRANK POSITION SENSOR — 11
AIR FLOWMETER — 20
INTAKE AIR TEMPERATURE SENSOR — 21
OXYGEN SENSOR — 25
THROTTLE POSITION SENSOR — 24
WATER TEMPERATURE SENSOR — 28
OPERATION SWITCH — 29

INPUT INTERFACE — 45

IGNITER — 14
THROTTLE MOTOR — 23
INJECTOR — 27
WARNING LAMP — 30

OUTPUT INTERFACE — 46

RAM — 44
ACCUMULATION COUNTER — 48
TRAVEL DISTANCE COUNTER — 49
DURATION COUNTER — 50

ROM — 43
UPPER LIMIT STORAGE AREA — 51

TIMER — 31
CPU — 42

41
47
CA

# FIG.3

SLUDGE PRECURSOR ACCUMULATION RATE [ppm / kgfuel]

WATER TEMPERATURE [°C]

32

5a 4a 3a 2a a 0

# FIG.4

```
                    ( START )
                        │
        ┌───────────────┤
        │               ▼
        │      ┌──────────────────────┐
  S1 ───┼──────│ OBTAIN DURATION AFTER │
        │      │ CHANGE OF ENGINE OIL  │
        │      └──────────────────────┘
        │               │
        │          S2   ▼
        │        ╱─────────────╲           YES
        │       ╱  HAS COUNT    ╲──────────────┐
        │      ╱ VALUE REACHED UPPER╲           │
        │       ╲     LIMIT?     ╱              │
        │        ╲─────────────╱               │
        │              │ NO                     │
        │              ▼                        │
        │      ┌──────────────────────┐         │
  S3 ───┼──────│ OBTAIN TRAVEL DISTANCE │        │
        │      │ AFTER CHANGE OF ENGINE OIL│     │
        │      └──────────────────────┘         │
        │              │                        │
        │         S4   ▼                         │
        │        ╱─────────────╲       YES        │
        │       ╱  HAS COUNT    ╲──────────────────▶
        │      ╱ VALUE REACHED UPPER╲              │
        │       ╲     LIMIT?     ╱                 │
        │        ╲─────────────╱                  │
        │              │ NO                        │
        │              ▼                           │
        │      ┌──────────────────────┐            │
  S5 ───┼──────│ PROCESSING FOR CALCULATING│        │
        │      │  ACCUMULATION AMOUNT     │        │
        │      │  OF SLUDGE PRECURSOR     │        │
        │      └──────────────────────┘            │
        │              │                           │
        │         S6   ▼                            │
        │        ╱─────────────╲                    │
        │  NO   ╱     HAS        ╲                   │
        └──────╱  ACCUMULATION    ╲                 │
               ╲ AMOUNT OF SLUDGE ╱                 │
                ╲PRECURSOR REACHED╱                 │
                 ╲  UPPER LIMIT? ╱                  │
                  ╲─────────────╱                   │
                        │ YES                        │
                        ▼◀──────────────────────────┘
               ┌──────────────────────┐
  S7 ──────────│ LIGHT OR BLINK WARNING LAMP│
               └──────────────────────┘
                        │
                        ▼
                    (  END  )
```

## INTERNATIONAL SEARCH REPORT

International application No.

PCT/JP2011/005894

### A. CLASSIFICATION OF SUBJECT MATTER
*F01M11/10*(2006.01)i

According to International Patent Classification (IPC) or to both national classification and IPC

### B. FIELDS SEARCHED

Minimum documentation searched (classification system followed by classification symbols)
F01M11/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y<br>A | JP 2008-95562 A  (Toyota Motor Corp.),<br>24 April 2008 (24.04.2008),<br>entire text; all drawings<br>(Family: none) | 1,3,5-7<br>2,4 |
| Y | JP 2010-145107 A  (Nippon Soken, Inc.),<br>01 July 2010 (01.07.2010),<br>paragraph [0063]<br>(Family: none) | 1,3,5-7 |
| Y | WO 2009/004973 A1  (Honda Motor Co., Ltd.),<br>08 January 2009 (08.01.2009),<br>paragraphs [0003] to [0004]<br>& JP 2009-8058 A        & JP 2009-8059 A<br>& US 2010/0180671 A1    & EP 2161419 A1 | 6,7 |

☒  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search<br>    12 January, 2012 (12.01.12) | Date of mailing of the international search report<br>    24 January, 2012 (24.01.12) |
| Name and mailing address of the ISA/<br>    Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2011/005894 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2010-77828 A  (Toyota Motor Corp.), 08 April 2010 (08.04.2010), paragraph [0004] (Family: none) | 1 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004156455 A